# EUROPÄISCHE PATENTSCHRIFT

(11) **EP 1 191 920 B1**
(45) Veröffentlichungstag und Bekanntmachung des Hinweises auf die Patenterteilung: **30.03.2005**
(21) Anmeldenummer: 99941555.7
(22) Anmeldetag: 10.08.1999
(51) Int. Cl.: A61K 7/00, A61K 7/09

(54) **VERFAHREN ZUR DAUERHAFTEN VERFORMUNG KERATINISCHER FASERN UND MITTEL**
METHOD FOR PERMANENTLY SHAPING KERATIN FIBRES, AND AGENTS
PROCEDE POUR L'ONDULATION PERMANENTE DE FIBRES KERATINIQUES, ET PRODUITS

(30) Priorität: 03.07.1999 DE 19930769
(43) Veröffentlichungstag der Anmeldung: 03.04.2002
(73) Patentinhaber: Hans Schwarzkopf & Henkel GmbH & Co. KG, 22763 Hamburg (DE)
(72) Erfinder: MÜLLER, Burkhard, D-21075 Hamburg (DE); KNAPPE, Thorsten, D-22525 Hamburg (DE); MANNECK, Hartmut, D-23860 Klein Wessenberg (DE); BICHELS, Dirk, D-40489 Düsseldorf (DE)
(74) Vertreter: Strohe-Kamp, Geertje
(86) Internationale Anmeldenummer: PCT/EP1999/005785
(87) Internationale Veröffentlichungsnummer: WO 1999/058099

(56) Entgegenhaltungen:
- DE-A- 3 009 763
- DE-C- 19 534 723
- US-A- 5 093 113
- PATENT ABSTRACTS OF JAPAN vol. 009, no. 218 (C-301), 5. September 1985 (1985-09-05) & JP 60 081117 A (RAION KK), 9. Mai 1985 (1985-05-09)

## Beschreibung

Die Erfindung betrifft ein Verfahren zur dauerhaften Verformung von Keratinfasern, insbesondere von menschlichen Haaren, durch reduktive Spaltung und erneute oxidative Knüpfung von Disulfidbindungen des Keratins sowie für dieses Verfahren geeignete Mittel.

Die dauerhafte Verformung von Keratinfasem wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar.

Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist aber häufig ein Verspröden und Stumpfwerden der Haare. Weiterhin werden in vielen Fällen auch andere Eigenschaften wie Naß- und Trockenkämmbarkeit, Griff, Geschmeidigkeit, Weichheit, Glanz und Reißfestigkeit in ungewünschter Weise beeinflußt.

Es hat daher in der Vergangenheit nicht an Versuchen gefehlt, hier für Abhilfe zu sorgen.

Eine entsprechende Modifizierung der reduzierenden Lösung führt zu in der Regel nicht befriedigenden Welleistungen. Die Zugabe bekannter Zusätze wie Strukturanten, Polymere, Filmbildner und vernetzende Harze oder die neutrale bis schwach saure Einstellung der Zubereitung kann die Schädigung des Haares zwar verringern, jedoch bleibt das Haar in seiner Struktur mehr oder weniger geschwächt Die pflegende Behandlung der Haare durch weitere Nachbehandlungen kann zwar die Haareigenschaften wieder verbessern, erfordert jedoch zusätzlichen Zeitaufwand und in der Regel die Verwendung mindestens eines weiteren Mittels.

Es bestand daher weiterhin die Aufgabe, ein Verfahren der dauerhaften Verformung von Keratinfasem zu finden, bei welchem die genannten unerwünschten Nebenwirkungen weiter reduziert oder ganz ausgeschlossen werden.

Es wurde nun überraschenderweise gefunden, daß eine wesentliche Verbesserung der Eigenschaften verformter Keratinfasern, wie verbesserte Kämmbarkeit und Avivage, dadurch erreicht wird, daß mindestens ein während des Verformungsverfahrens eingesetztes Mittel mehrphasig formuliert ist und spezielle Verbindungen enthält.

Gegenstand der Erfindung ist daher ein Verfahren zur dauerhaften Verformung von Keratinfasem, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült und gegebenenfalls nachbehandelt, dadurch gekennzeichnet, daß mindestens eine der beiden wäßrigen Zubereitungen oder die erste Spülung in Form eines Zwei- oder Mehrphasensystems, enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenfalls mindestens eine Ölkomponente enthält und das zum Aufbringen auf die Fasser durch mechanische Bewegung in ein homogenes System überführt wird.

Im weiteren werden folgende Bezeichnungen verwendet:
- "Wellmittel" für die wäßrige Zubereitung der keratinreduzierenden Substanz,
- "Zwischenspülung" für die erste Spülung und
- "Fixiermittel" für die wäßrige Zubereitung des Oxidationsmittels.

Im erfindungsgemäßen Verfahren ist das Wellimitiel, die Zwischenspülung und/oder das Fixiermittel in Form eines Zwei- oder Mehrphasensystems formuliert. Erfindungsgemäß eingesetzte Zwei- und Mehrphasensysteme sind Systeme, bei denen mindestens zwei separate, kontinuierliche Phasen vorliegen. Beispiele für solche Systeme sind Zubereitungen, die folgende Phasen aufweisen:
- eine wäßrige Phase und eine nichtwäßrige Phase, die separat voneinander vorliegen
- eine wäßrige Phase und zwei nichtwäßrige, miteinander nicht mischbare Phasen, die jeweils separat vorliegen
- eine Öl-in-Wasser-Emulsion und eine davon separiert vorliegende nichtwäßrige Phase
- eine Wasser-in-Öl-Emulsion und eine davon separiert vorliegende wäßrige Phase.

Keine Zwei-Phasensysteme im Sinne der vorliegenden Erfindung sind Systeme, bei denen nur eine kontinuierliche Phase vorliegt, wie z. B. reine Öl-in-Wasser- oder Wasser-in-Öl-Emulsionen.

Die im erfindungsgemäßen Verfahren eingesetzten, Zwei- oder Mehrphasensysteme aufweisenden Mittel entfalten ihre volle Wirkung aber nur dann, wenn sie in homogener Form auf die Keratinfaser aufgebracht werden. Dazu werden die Mittel durch mechanische Einwirkung, z. B. einfaches Schütteln des sie enthaltenden Behälters mit der Hand, in homogene Systeme überführt. Um ein homogenes Aufragen auf. die Keratinfaser sicherzustellen, muß dieser homogene Zustand für eine ausreichende Zeit erhalten bleiben, bevor sich die einzelnen Phasen wieder ausbuden. Für die erfindungsgemäße Lehre hat es sich als ausreïchend erwiesen, wenn dieser homogene Zustand mindestens 20 Sekunden, insbesondere mindestens 30 Sekunden, stabil ist, bevor für den Betrachter wieder eine Grenzschicht und somit die Ausbildung der einzelnen Phasen erkennbar wird.

Die erfindungsgemäß eingesetzten Zwei- und Mehrphasensysteme enthalten neben Wasser zwingend mindestens einen mit Wasser nur begrenzt mischbaren Alkohol mit 4 bis 10 C-Atomen.

Erfindungsgemäß geeignete Ölkomponenten sind prinzipiell alle Öle und Fettstoffe sowie deren Mischungen mit festen Paraffinen und Wachsen. Bevorzugt sind solche Ölkomponenten, deren Löslichkeit in Wasser bei 20 °C kleiner Gew -%, insbesondere kleiner als 0,1 Gew.-% beträgt. Der Schmelzpunkt der einzelnen Öl- oder Fettkomponenten liegt bevorzugt unterhalb von etwa 40 °C. Ölkomponenten, die bei Raumtemperatur, d.h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Aprikosenkernöl, Avokadoöl, Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

Eine weitere, besonders bevorzugte Gruppe erfindungsgemäß einsetzbarer Ölkomponenten sind flüssige Paraffinöle und synthetische Kohlenwasserstoffe sowie Din-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-nundecylether, Di-n-dodecylether, n-Hexyl-n-octylether, n-Octyl-n-decylether, n-Decyl-nundecylether, n-Undecyl-n-dodecylether und n-Hexyl-n-Undecylether sowie Di-tertbutylether, Di-iso-pentylether, Di-3-ethyldecylether, tert.-Butyl-n-octylether, iso-Pentyln-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Glycerin, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-diisostearat und Neopentylglykoldi-capylat erfindimgsgemäß verwendbare Ölkomponenten dar, ebenso komplexe Ester wie z. B. das Diacetyl-glycerinmonostearat.

Erfindüngsgemäß verwendbare, ebenfalls bevorzugte Ölkomponenten sind schließlich auch Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067. (Hersteller: Wacker) sowie Abil® -Quat 3270 und 3272 (Hersteller: Th. Goldschmidt; diquatemäre Polydimethylsiloxane, Quaternium-80).

Erfindungsgemäß einsetzbare Ölkomponenten sind schließlich auch Dialkylcarbonate, wie sie in der DE-OS 197 101 54, auf die ausdrücklich Bezug genommen wird, ausführlich beschrieben werden. Dioctylcarbonate, insbesondere das Di-2-ethylhexylcarbonat, sind bevorzugte Ölkomponenten in Rahmen der vorliegenden Erfindung.

Unter "mit Wasser begrenzt mischbar" werden solche Alkohole verstanden, die in Wasser bei 20 °C zu nicht mehr als 10 Gew.-%, bezogen auf die Wassermasse, löslich sind. In vielen Fällen haben sich Triole und insbesondere Diole als erfindungsgemäß besonders geeignet erwiesen. Die erfindungsgemäß verwendeten Alkohole mit 4 bis 10 Kohlenstoffatomen gesättigt oder ungesättigt und linear, verzweigt oder cyclisch sein. Einsetzbar im Sinne der Erfindung sind beispielsweise Butanol-1, Cyclohexanol, Pentanol-1, Decanol, Octanol, Octenol, Decenol, Octadienol und Decadienol, wobei diese Aufzählung beispielhaften und nicht limitierenden Charakter haben soll.

Erfindungsgemäß bevorzugt als Alkohole mit 4 bis 10 Kohlenstoffatomen sind 2-Ethyl-hexandiol-1,3, Butanol-1, Cyclohexanol, Pentanol-1 und 1,2-Butandiol. Insbesondere 2-Ethyl-hexandiol-1,3, aber auch Butanol-1 und Cyclohexanol sind besonders bevorzugt.

Die wäßrigen und die nichtwäßrigen Phasen liegen in den in dem erfindungsgemäßen Verfahren verwendeten Mitteln in Mengenverhältnissen (bezogen auf das Gewicht) von 1:200 bis 1:1, bevorzugt von 1 :40 bis 1:5 und besonders bevorzugt von 1:20 bis 1:10 vor. In Fällen, in denen mehrere nichtwäßrige Phasen vorliegen, beziehen sich diese Angaben auf die Gesamtheit der nichtwäßrigen Phasen.

Die erfindungsgemäße Lehre umfaßt auch solche Ausführungsformen des erfindungsgemäßen Verfahrens, bei denen das mehrphasige Mittel aus zwei oder mehr getrennt konfektionierten Ausgangszubereitungen erst unmittelbar vor der Anwendung hergestellt wird. Diese Ausführungsform kann bei extrem inkompatiblen Bestandteilen bevorzugt sein.

Ein weiterer Gegenstand der Anmeldung sind zur Durchführung des erfindungsgemäßen Verfahrens verwendete Mittel.

Diese Mittel dienen im Rahmen eines Verfahrens zur dauerhaften Verformung von Keratinfasern entweder zur Durchführung der reduzierenden Stufe oder zur Durchführung der oxidierenden Stufe und können prinzipiell alle für diese Mittel üblichen Bestandteile enthalten, sofern die erfindungsgemäßen Bedingungen (Vorliegen des Zwei- oder Mehrphasensystems und die kurzzeitige Mischbarkeit) gegeben sind.

Bevorzugt wird das erfindungsgemäße Verfahren zum Dauerwellen bzw. Glätten von menschlichen Haaren verwendet.

Gemäß einer bevorzugten Ausfühmngsform des erfindungsgemäßen Verfahrens ist die Wellotion in Form des oben genannten Zwei- oder Mehrphasensystems formuliert. Es hat sich überraschenderweise gezeigt, daß auf diese Weise formulierte Wellotionen bei gleicher Menge der jeweiligen keratinreduzierenden Komponenten einen deutlich erhöhten Welleffekt ergeben. Analog kann die mit einer nicht erfindungsgemäß formulierten Wellotion erzielte Welleistung mit einer erfindungsgemäß formulierten Wellotion unter deutlicher Verringerung des Anteils der keratinreduzierenden Substanz, und somit unter Schonung von Haar und Kopfhaut, erreicht werden.

Weiterhin hat sich gezeigt, daß durch Formulierung der Wellotion als Zwei- und Mehrphasensystem das Problem der Parfümierung deutlich verringert werden kann. Aufgrund der von den meisten Anwendern nicht tolerierten Duftnote der in der Wellotion zwingend erforderlichen Bestandteile (keratinreduzierende Thio-Verbindungen, ggf. Alkalien wie Ammoniak oder Alkanolamine) ist eine Parfümierung jedoch praktisch unerläßlich. Problematisch ist, daß die überwiegende Zahl der Parfümkomponenten in diesen Wellotionen nicht lagerstabil ist. Somit ist die Auswahl der Duftnoten für solche Mittel stark eingeschränkt. Es hat sich nun ebenfalls überraschenderweise gezeigt, daß bei Verwendung der erfindungsgemäß definierten Zwei- oder Mehrphasensysteme eine Vielzahl weiterer Parfümkomponenten in diese Wellotion lagerstabil eingearbeitet werden kann. Auch in erfindungsgemäßen aufgebauten Fixierlösungen haben sich zusätzliche Parfümkomponenten als nun lagerstabil einarbeitbar erwiesen. Bei Zwischenspülungen mit den erfindungsgemäßen Zwei- und Mehrphasensystemen hat es sich erwiesen, daß in vielen Fällen auf die Verwendung von Emulgatoren und Lösungsvermittlern für die Einarbeitung der Parfumkomponenten verzichtet werden kann.

Ein zweiter Gegenstand der Erfindung ist daher ein Mittel zur Durchführung der reduzierenden Stufe eines Verfahrens zur dauerhaften Verformung von Keratinfäsern enthaltend eine keratinreduzierende Substanz sowie übliche Bestandteile, dadurch gekennzeichnet, daß es in Form eines Zwei- oder Mehrphasensystems,

Die erfindungsgemäßen Wellmittel enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet sind die Alkali- oder Ammoniumsalze der Thioglykolsäure und/oder der Thiömilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammoniumcarbonate und -hydrogencarbonate oder organische Amine wie Monoethanolamin.

Weiterhin können die erfindungsgemäßen Wellotionen wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen wie beispielsweise die enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenenfalls mindestens eine Ölkomponente enthält und das durch mechanische Bewegung in ein homogenes System überführt werden kann.
   C₁₋₄ -Alkyl-Derivate, C₁₋₄-Hydroxyalkyl-Derivate und C₁₋₄ -Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Die Aminosäuren können sowohl als freie Aminosäure als auch als Salze, z. B. als Hydrochloride, eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.

Bezüglich näherer Informationen zu solchen wellkraftverstärkenden Komponenten wird auf die Druckschriften DE-OS 44 36 065 und EP-B 1-363 057 verwiesen, auf deren Inhalt ausdrücklich Bezug genommen wird.

Die wellkraftverstärkenden Verbindungen können in den erfindungsgemäßen Wellotionen in Mengen von 0,5 bis 5 Gew.-%, bezogen auf die gesamte Wellotion, enthalten sein. Mengen von 1 bis 4 Gew.-%, im Falle der Diole von 0,5 - 3 Gew.-%, haben sich als ausreichend erwiesen, weshalb diese Mengen besonders bevorzugt sind.

Ein dritter. Gegenstand der Erfindung ist ein Mittel zur Durchführung der oxidierenden Stufe eines Verfahrens zur dauerhaften Verformung von Keratinfasern enthaltend ein Oxidationsmittel sowie übliche Bestandteile, dadurch gekennzeichnet, daß es in Form eines Zwei- oder Mehrphasensystems, enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenenfalls mindestens eine Ölkomponente enthält und das durch mechanische Bewegung in ein homogenes System überführt werden kann.

Zwingender Bestandteil der erfindungsgemäßen Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 15 Gew.-%, gebrauchsfertig in der Regel etwa 0,5 - 3 Gew.-%, H₂O₂ enthalten, liegt bevorzugt bei 2 bis 6, insbesondere 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere H₂O₂, enthalten.

Weiterhin hat es sich als vorteilhaft erwiesen, wenn die erfindungsgemäßen Mittel einen pflegenden Wirkstoff, ausgewählt aus Proteinhydrolysaten und deren Derivaten, enthalten.

Geeignete Proteinhydrolysate sind insbesondere Elastin-, Kollagen-, Keratin-, Milcheiweiß-, Seidenprotein-, Sojaprotein-, Mandelprotein-, Erbsenprotein-, Kartoffelprotein-, Haferprotein-, Maisprotein- und Weizenproteinhydrolysate. Dabei können Produkte auf pflanzlicher Basis erfindungsgemäß bevorzugt sein.

Geeignete Derivate der Proteinhydrolysate sind insbesondere deren Kondensationsprodukte mit Fettsäuren und Fettsäuregemischen, wie Ölsäure, Myristinsäure, Undecylensäure, Kokosfettsäure und Abietinsäure. Die Kondensationsprodukte können auch in Form von Salzen, insbesondere Natrium-, Kalium- und Triethanolaminsalzen, vorliegen.

Ebenfalls geeignete Derivate sind quaternisierte Proteinhydrolysate. Beispiele für diese Verbindungsklasse sind die unter den Bezeichnungen Lamequat® L(CTFA-Bezeichnung: Lauryldimonium Hydroxypropylamino Hydrolyzed Animal Protein; Grünau), Croquat® WKP und Gluadin® WQ auf dem Markt befindlichen Produkte. Das letztgenannte Produkt, das auf pflanzlicher Basis beruht, kann bevorzugt sein.
Die Protein-Derivate sind in den erfindungsgemäßen Mitteln bevorzugt in Mengen von 0,1 bis 10 Gew.-%, bezogen auf die Gesamtmenge des Mittels enthalten. Mengen von 0,1 bis 5 Gew.-% sind bevorzugt.

Bevorzugt enthalten die erfindungsgemäßen Mittel weiterhin mindestens einen konditionierenden Wirkstoff.

Als konditionierende Wirkstoffe kommen bevorzugt kationische Polymere in Betracht. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate.
- Polysiloxane mit quaternären Gruppen,
- Polymere Dimethyldiallylammorvumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammo-niumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat® 755 im Handel erhältlich.
- Vinylpyrrolidon-Vinylimidazoliniummethochlorid-Copolymere, wie sie unter der Bezeichnung Luviquat® angeboten werden,
- quaternierter Polyvinylalkohol
sowie die unter den Bezeichnungen
- Polyquaternium 2,
- Polyquaternium 17,
- Polyquaternium 18 und
- Polyquaternium 27
bekannten Polymeren mit quartären Stickstoffatomen in der Polymerhauptkette.

Geeignet als konditionierende Wirkstoffe sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Gruppe enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat® 2001 N im Handel erhältlich sind sowie das Handelsprodukt Merquat® 280, sind erfindungsgemäß besonders bevorzugte Ampho-Polymere.

Die kationischen oder amphoteren Polymere sind in den erfindungsgemäßen Zubereitungen bevorzugt in Mengen von 0,1 bis 5 Gew.-%, bezogen auf die gesamte Zubereitung, enthalten.

Als konditionierende Wirkstoffe geeignete Silikonöle und Silikon-Gums sind insbesondere Dialkyl- und Alkylarylsiloxane, wie beispielsweise Dimethylpolysiloxan und Methylphenylpolysiloxan, sowie deren alkoxylierte und quatemierte Analoga. Beispiele für solche Silikone sind die von Dow Coming unter den Bezeichnungen DC 190, DC 200 und DC 1401 vertriebenen Produkte sowie das Handelsprodukt Fancorsil® LIM-1.

Erfindungsgemäß als konditionierende Wirkstoffe ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning® 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80). Ein geeignetes anionisches Silikonöl ist das Produkt Dow Corning® 1784.

Beispiele für die in den erfindungsgemäßen Mitteln als konditionierende Wirkstoffe verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, sogenannte "Esterquats", wie beispielsweise die unter den Warenzeichen Dehyquart® und Stepantex® vertriebenen Methyl-hydroxyalkyldialkoyloxyalkyl-ammoniummethosulfate, eingesetzt werden.

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Weiterhin kann es bevorzugt sein, die einzelnen Phasen mit Farbstoffen anzufärben, um ein besonders gutes optisches Erscheinungsbild des Mittels zu erzielen. Diese Farbstoffe sind bevorzugt nur in der wäßrigen oder nur in mindestens einer nichtwäßrigen Phase in einer Menge löslich, die eine entsprechende Einfärbung für den Betrachter sichtbar erscheinen läßt. Es ist auch möglich, sowohl die nichtwäßrige als auch die wäßrige Phase mit verschiedenen Farbstoffen, bevorzugt in verschiedenen Farben, einzufärben. Das alleinige Anfärben einer nichtwäßrigen Phase ist jedoch bevorzugt.

Weitere übliche Bestandteile für die erfindungsgemäßen Mittel sind:
- anionische Tenside wie beispielsweise Seifen, Alkylsulfate und Alkylpolyglykolethersulfate, Salze von Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure Alkylglykosiden oder Alkoholen, welche Anlagerungsprodukte von etwa 2-15 Molekülen Ethylenoxid und/oder Propylenoxid an Fettalkohole mit 8 bis 22 C-Atomen darstellen.
- zwitterionische Tenside wie beispielsweise Betaine und 2-Alkyl-3-carboxymethyl-3-hydroxyethyl-imidazoline.
- ampholytische Tenside, wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren.
- nichtionische Tenside wie beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmonound -oligoglycoside und deren ethoxylierte Analoga sowie Anlagerungsprodukte von 5 bis 60 Mol Ethylenoxid an Rizinusöl und gehärtetes Rizinusöl.
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden, Polyoxycarbonsäuren, wie Polyketo- und Polyaldehydocarbonsäuren und deren Salze,
   sowie Polymere und Copolymere der Crotonsäure mit Estern und Amiden der Acrylund der Methacrylsäure, wie Vinylacetat-Crotonsäure- und Vinylacetat-Vinylpropionat-Crotonsäure-Copolymere,
- organische Verdickungsmittel wie Agar-Agar, Guar-Gum, Alginate, Celluloseether, wie Methyl- und Methylhydroxypropylcellulose, Gelatine, Pektine und/oder Xanthan-Gum. Ethoxilierte Fettalkohole, insbesondere solche mit eingeschränkter Homologenverteilung, wie sie beispielsweise als Handelsprodukt unter de-Bezeichnung Arlypon® F (Henkel) auf dem Markt sind, alkoxylierte Methylglucosidester, wie das Handelsprodukt Glucamate® DOE 120 (Amerchol), und ethoxylierte Propylenglykolester, wie das Handelsprodukt Antil® 141 (Goldschmidt), können bevorzugte organische Verdickungsmittel sein.
- Strukturanten wie Glucose und Maleinsäure,
- haarkonditionierende Verbindungen wie Phospholipide, beispielsweise Sojalecithin, Ei-Lecithin und Kephaline,
- Parfümöle,
- Lösungsvermittler, wie Ethanol, Isopropanol, Ethylenglykol, Propylenglykol, Glycerin, Diethylenglykol und ethoxylierte Triglyceride sowie Fettalkoholethoxylate und deren Derivate,
- Antischuppenwirkstoffe wie Climbazol, Piroctone Olamine und Zink Omadine,
- Wirkstoffe wie Bisabolol, Allantoin, Panthenol, Niacinmid, Tocopherol und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe sowie
- Treibmittel wie Propan-Butan-Gemische, N₂O, Dimethylether, CO₂ und Luft.

Bezüglich der weiteren Bestandteile der erfindungsgemäßen Mittel und deren übliche Einsatzmengen wird ausdrücklich auf die bekannten Monographien, z. B. Umbach, Kosmetik, 2. Auflage, Georg Thieme Verlag, Stuttgart New York, 1995, und Kh Schrader, Grundlagen und Rezepturen der Kosmetika, 2. Auflage, Hüthig Buch Verlag, Heidelberg, 1989, verwiesen.

Die folgenden Beispiele sollen die Erfindung näher erläutern.

### Beispiele

### Soweit nicht anders vermerkt, sind alle Angaben Gewichtsteile

### 1. Wellmittel (2 Phasen)

| | |
|---|---|
| Ammoniumthioglykolat (71 %ig in Wasser) | 16,0 |
| Ammoniumhydrogencarbonat | 9,0 |
| Lamepon® S | 1,0 |
| Merquat® 100 | 0,5 |
| Gluadin® WQ | 0,5 |
| 2-Ethylhexandiol-1,3 | 5,0 |
| Farbstoff | 0,0002 |
| Parfümöl | 1,0 |
| Ammoniak (25 %ig in Wasser) | ad pH 8,4 |
| Wasser | ad 100 |

Die Weil-Lotion erzeugte eine intensive, gleichmäßige Welle mit hoher Sprungkraft. Die Haare waren nach der Behandlung sehr gepflegt und gut kämmbar.

### 2. Fixiermittel (3 Phasen)

| | |
|---|---|
| Wasserstoffperoxid (50 %ig in Wasser) | 4,0 |
| Aromox® MCD-W¹⁰ | 1,0 |
| Turpinal® SL¹¹ | 1,0 |
| Dioctylcarbonat | 5,0 |
| Paraffinöl | 2,0 |
| 2-Ethylhexandiol-1,3 | 5,0 |
| Wasser | 82,0 |

| | |
|---|---|
| ¹⁰ N,N-Dimethyl-N-kokosalkylamin-N-oxid (30 % Aktivsubstanz in Wasser; INCI-Bezeichnung: Cocamine Oxide) (AKZO) | |
| ¹¹ 1-Hydroxyethan- 1,1 -diphosphonsäure (ca. 60 % Aktivsubstanz in Wasser; INCI-Bezeichnung: Etidronic Acid) (HENKEL) | |

Mit Hilfe der erfindungsgemäßen Fixierung war das Haar nach der Behandlung sehr gut kämmbar und wirkte sehr gepflegt.

### 3. Wellmittel für poröses Haar (2-Phasen)

| | |
|---|---|
| Ammoniumthioglykolat (71%ig in Wasser) | 10,0 |
| Ammoniumhydrogencarbonat | 3,0 |
| Lamepon® S | 1,0 |
| Merquat® 100 | 0,5 |
| Gluadin® WQ | 0,5 |
| 2-Ethylhexandiol-1,3 | 4,0 |
| Farbstoff | 0,0001 |
| Parfümöl | 1,0 |
| Imidazol | 5,0 |
| Wasser | ad 100 |

Die Well-Lotion erzeugte auf porösem Haar eine intensive, gleichmäßige Welle mit hoher Sprungkraft. Die Haare waren nach der Behandlung sehr gepflegt und gut kämmbar.

### 4. Mehrkomponenten-Wellmittel (2-Phasen)

| Komponente A (2-Phasen) | |
|---|---|
| Ammoniumbicarbonat | 6,0 |
| Ammoniak | 1,0 |
| 2-Ethylhexandiol-1,3 | 10,0 |
| Parfüm | 1,0 |
| Merquat® 100 | 0,1 |
| Croquat® WKP¹² | 0,1 |
| Wasser | ad 100 |

| | |
|---|---|
| ¹² quatemisiertes Keratinhydrolysat (ca. 32 % Aktivsubstanz in Wassser; INCI-Bezeichnung: Cocodimonium Hydroxypropyl Hydrolyzed Keratin) (CRODA) | |

| Komponente B | |
|---|---|
| Ammoniumthioglykolat (71 %ig in Wasser) | 50 |
| Ammoniumthiolactat (70% in Wasser) | 25 |
| Wasser | ad 100 |

Vor Gebrauch werden 52 ml der Komponente A mit 23 ml der Komponente B vermischt. Es entsteht eine gebrauchsfertige Mischung, die nur vorübergehend homogen ist, sich jedoch nach einigen Minuten sichtbar in zwei Phasen trennt. Die Weil-Lotion erzeugte eine intensive, gleichmäßige Welle mit hoher Sprungkraft. Die Haare waren nach der Behandlung sehr gepflegt und gut kämmbar.

### 5. Wärmeaktiviertes 2-Komponenten-Wellmittel für poröses Haar (2-Phasen)

| Komponente A (2-Phasen) | |
|---|---|
| Ammoniumthioglykolat (71%ig in Wasser) | 21,7 |
| Ammoniak | 2,8 |
| 2-Ethylhexandiol-1,3 | 8,0 |
| Parfüm | 1,0 |
| Merquat® 100 | 2,5 |
| Lamepon® S | 1,0 |
| Wasser | ad 100 |

| Komponente B | |
|---|---|
| Wasserstoffperoxid 50% | 7,2 |
| Phosphorsäure 85% | 0,15 |
| PHB-Methylester | 0,04 |
| Wasser | ad 100 |

Vor Gebrauch werden 60 ml der Komponente A mit 15 ml der Komponente B vermischt. Es entsteht eine gegenüber vor Vermischen um 15 - 20°C erwärmte, gebrauchsfertige Mischung, die nur vorübergehend homogen ist, sich jedoch nach einigen Minuten sichtbar in zwei Phasen trennt. Die Weil-Lotion erzeugte auf porösem Haar eine intensive, gleichmäßige Welle mit hoher Sprungkraft. Die Haare waren nach der Behandlung sehr gepflegt und gut kämmbar.

## Patentansprüche

1. Verfahren zur dauerhaften Verformung von Keratinfasern, bei welchem man die Fasser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült und gegebenenfalls nachbehandelt, **dadurch gekennzeichnet, daß** mindestens eine der beiden wäßrigen Zubereitungen oder die erste Spülung in Form eines Zwei- oder Mehrphasensystems, enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenfalls mindestens eine Ölkomponente enthält und das zum Aufbringen auf die Faser durch mechanische Bewegung in ein homogenes System überführt wird.

2. Verfahren nach Anspruch 1, **dadurch gekennzeichnet, daß** die Ölkomponente ausgewählt ist aus pflanzlichen Ölen, Paraffinölen und Silikonen.

3. Verfahren nach einem der Ansprüche 1 oder 2, **dadurch gekennzeichnet, daß** der mit Wasser begrenzt mischbare Alkohol ein Diol oder Triol ist.

4. Verfahren nach einem der Ansprüche 1 bis 3, **dadurch gekennzeichnet, daß** der mit Wasser begrenzt mischbare Alkohol verzweigt ist.

5. Mittel zur Durchführung der reduzierenden Stufe eines Verfahrens zur dauerhaften Verformung von Keratinfasern enthaltend eine keratinreduzierende Substanz sowie übliche Bestandteile, **dadurch gekennzeichnet, daß** es in Form eines Zwei- oder Mehrphasensystems, enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenenfalls mindestens eine Ölkomponente enthält und das zum Aufbringen auf die Faser durch mechanische Bewegung in ein homogenes System überführt wird.

6. Mittel zur Durchführung der oxidierenden Stufe eines Verfahrens zur dauerhaften Verformung von Keratinfasern enthaltend ein Oxidationsmittel sowie übliche Bestandteile, **dadurch gekennzeichnet, daß** es in Form eines Zwei- oder Mehrphasensystems, enthaltend mindestens zwei separate, kontinuierliche Phasen, vorliegt, das mindestens einen mit Wasser nur begrenzt mischbaren Alkohol mit 4 bis 10 C-Atomen, der gesättigt oder ungesättigt und linear, verzweigt oder cyclisch sein kann, und gegebenfalls mindestens eine Ölkomponente enthält und das zum Aufbringen auf die Fasser durch mechanische Bewegung in ein homogenes System überführt wird.

7. Mittel nach einem der Ansprüche 5 bis 6, **dadurch gekennzeichnet, daß** es weiterhin einen Wirkstoff, ausgewählt aus Proteinhydralysaten und deren Derivaten, enthält.

8. Mittel nach einem der Ansprüche 5 bis 7, **dadurch gekennzeichnet, daß** ein konditionierender Wirkstoff enthalten ist.

## Claims

1. A process for the permanent deforming of keratin fibers in which the fibers are treated before and/or after mechanical deforming with an aqueous preparation of a keratin-reducing substance, rinsed with a first rinse after a certain contact time, fixed with an aqueous preparation of an oxidizing agent and rinsed and optionally aftertreated, again after a certain contact time, **characterized in that** at least one of the two aqueous preparations or the first rinse is present in the form of a two-phase or multiphase system containing at least two separate continuous phases which contains at least one saturated or unsaturated, linear, branched or cyclic C₄₋₁₀ alcohol having only limited miscibility with water and optionally at least one oil component and which, for application to the fibres, is converted by mechanical agitation into a homogeneous system.

2. A process as claimed in claim 1, **characterized in that** the oil component is selected from vegetable oils, paraffin oils and silicones.

3. A process as claimed in claim 1 or 2, **characterized in that** the alcohol having limited miscibility with water is a diol or triol.

4. A process as claimed in any of claims 1 to 3, **characterized in that** the alcohol having only limited miscibility water is branched.

5. A preparation for carrying out the reducing step of a process for permanently deforming keratin fibres containing a keratin-reducing substance and typical ingredients, **characterized in that** it is present in the form of a two-phase or multiphase system containing at least two separate continuous phases which contains at least one saturated or unsaturated, linear, branched or cyclic C₄₋₁₀ alcohol having only limited miscibility with water and optionally at least one oil component and which, for application to the fibres, is converted by mechanical agitation into a homogeneous system.

6. A preparation for carrying out the oxidizing step of a process for permanently deforming keratin fibers containing an oxidizing agent and typical ingredients, **characterized in that** it is present in the form of a two-phase or multiphase system containing at least two separate continuous phases which contains at least one saturated or unsaturated, linear, branched or cyclic C₄₋₁₀ alcohol having only limited miscibility with water and optionally at least one oil component and which, for application to the fibres, is converted by mechanical agitation into a homogeneous system.

7. A preparation as claimed in any of claims 5 to 6, **characterized in that** it additionally contains an active substance selected from protein hydrolyzates and derivatives thereof.

8. A preparation as claimed in any of claims 5 to 7, **characterized in that** it contains a conditioning agent.

## Revendications

1. Procédé pour l'ondulation permanente de fibres kératiniques, dans lequel on traite les fibres avant et/ou après une ondulation mécanique avec une composition aqueuse d'une substance réduisant la kératine, on lave avec un premier produit de rinçage après un temps d'action, puis on fixe avec une composition aqueuse d'un oxydant et on lave le cas échéant également après un temps d'action et on post-traite le cas échéant, **caractérisé en ce qu'**au moins une des deux compositions aqueuses ou le premier produit de rinçage se trouve sous forme d'un système à deux phases ou plus, contenant au moins deux phases séparées continues, qui contient au moins un alcool, qui n'est miscible que de manière limitée avec l'eau, comprenant 4 à 10 atomes de carbone, qui peut être saturé ou insaturé et linéaire, ramifié ou cyclique et le cas échéant au moins un composant huileux et qui est transformé en un système homogène par un mouvement mécanique en vue de l'application sur les fibres.

2. Procédé selon la revendication 1, **caractérisé en ce que** le composant huileux est choisi parmi les huiles végétales, les huiles de paraffine et les silicones.

3. Procédé selon l'une quelconque des revendications 1 ou 2, **caractérisé en ce que** l'alcool miscible de manière limitée avec l'eau est un diol ou un triol.

4. Procédé selon l'une quelconque des revendications 1 à 3, **caractérisé en ce que** l'alcool miscible de manière limitée avec l'eau est ramifié.

5. Agent pour réaliser l'étape de réduction d'un procédé en vue de l'ondulation permanente de fibres kératiniques, contenant une substance réduisant la kératine ainsi que des constituants usuels, **caractérisé en ce qu'**il se trouve sous forme d'un système à deux phases ou plus, contenant au moins deux phases séparées continues, qui contient au moins un alcool, qui n'est miscible que de manière limitée avec l'eau, comprenant 4 à 10 atomes de carbone, qui peut être saturé ou insaturé et linéaire, ramifié ou cyclique et le cas échéant au moins un composant huileux et qui est transformé en un système homogène par un mouvement mécanique en vue de l'application sur les fibres.

6. Agent pour réaliser l'étape d'oxydation d'un procédé en vue de l'ondulation permanente de fibres kératiniques, contenant un oxydant ainsi que des constituants usuels, **caractérisé** en ce en ce qu'il se trouve sous forme d'un système à deux phases ou plus, contenant au moins deux phases séparées continues, qui contient au moins un alcool, qui n'est miscible que de manière limitée avec l'eau, comprenant 4 à 10 atomes de carbone, qui peut être saturé ou insaturé et linéaire, ramifié ou cyclique et le cas échéant au moins un composant huileux et qui est transformé en un système homogène par un mouvement mécanique en vue de l'application sur les fibres.

7. Agent selon l'une quelconque des revendications 5 à 6, **caractérisé en ce qu'**il contient en outre une substance active, choisie parmi les hydrolysats de protéines et leurs dérivés.

8. Agent selon l'une quelconque des revendications 5 à 7, **caractérisé en ce qu'**il contient une substance active de conditionnement.
